# EUROPEAN PATENT APPLICATION

(11) **EP 1 942 352 A2**
(43) Date of publication of application: **09.07.2008**
(21) Application number: 08000059.9
(22) Date of filing: 03.01.2008
(51) Int. Cl.: G01S 7/52

(54) **Ultrasonic diagnostic equipment and method for processing signal of ultrasonic diagnostic equipment**

(30) Priority: 05.01.2007 JP 2007000778; 29.08.2007 JP 2007223257
(71) Applicant: Olympus Medical Systems Corp., Tokyo 151-0072 (JP)
(72) Inventor: Kozai, Shigenori, Tokyo 151-0072 (JP)
(74) Representative: Wibbelmann, Jobst

(57) **Abstract**

In the present invention, an image processing device (5) is configured by including an ultrasonic wave transmitting section (11), an ultrasonic wave receiving section (12), a transmission and reception control section (13), a B mode signal processing section (14), a B mode image generating and storing section (15), an image synthesizing section (16), a Doppler signal processing section (18), a Doppler image generating and storing section (15), a Doppler scan region setting section (17), a Doppler scan region storing section (20), a Doppler scan control section (22), a region marker movement control section (23) and a region marker image generating section (24). By the configuration, an observation target of which blood state needs to be grasped can be always optimally observed in real time in a Doppler image by a Color Doppler mode or a Power Doppler mode.

## Description

### BACKGROUND OF THE INVENTION

### 1. Field of the Invention

The present invention relates to ultrasonic diagnostic equipment and a method for processing a signal of ultrasonic diagnostic equipment which displays a blood flow image in a Color Doppler mode or a Power Doppler mode by ultrasonic waves in part of a B mode image display region.

### 2. Description of the Related Art

In recent years, in ultrasonic diagnostic equipment for medical use, ultrasonic waves are irradiated to a living body, and two-dimensional distribution of a blood flow is displayed in a color image by the power of a Doppler signal. Unlike the method for displaying a blood flow by the frequency of a Doppler signal, the direction and velocity of a blood flow, or velocity dispersion or the like cannot be displayed with the method for displaying a blood flow by the power of a Doppler signal. However, the method for displaying a blood flow by the power of a Doppler signal is one of useful blood flow display methods in view of the unique function of being able to display the location and intensity of the blood flow with high sensitivity and a high S/N ratio.

In the equipment of this kind, as described in, for example, Non-patent Document 1 "Diagnostic Imaging, December 1993, p. 66-69", a blood flow display region is provided in part of a B mode image display region, so that a blood flow image is displayed in the blood flow display region. The blood flow display region can be freely moved in the B mode image display region by an operating device, whereby the blood flow image of a desired region of interest can be displayed.

A blood flow image is displayed in a color corresponding to the power of the Doppler signal, and is displayed in, for example, violet, red, orange and yellow colors in sequence of increase in power. The portion without a blood flow has no color display, specifically, a B mode image is displayed, so that the portion without a blood flow can be clearly distinguished from the portion with a blood flow. The blood flow image by the power of the Doppler signal has a favorable S/N ratio, and therefore, the B mode image is displayed for the portion without a blood flow.

Therefore, when a tumor image is displayed in the B mode image display region on a display surface of an image display device and when a blood flow display region is moved to a part where the tumor image is present to display the blood flow of the portion, if the blood flow display region lies on the tumor image while the blood flow display region is moving, the tumor image is hidden and becomes invisible, and the target cannot be confirmed.

Generally, an ultrasonic transducer, for example, an ultrasonic probe is caused to abut on a subject by an operator, and therefore, its orientation is easily changeable. Therefore, the tumor image always has to be kept to be displayed on the display surface by correcting the deviation of the orientation of the echo sounder transducer while watching the screen, but if the target becomes invisible partway as described above, the deviation cannot be corrected if the orientation of the echo sounder transducer deviates. Accordingly, it becomes uncertain whether the blood flow displayed in the blood flow display region is surely the blood flow of the desired part.

Thus, for example, Japanese Patent Laid-Open No. 8-173428 or the like proposes ultrasonic diagnostic equipment which makes at least a B mode image observable even while the flow display region is moved by displaying the B mode image in the blood flow display region even while the blood flow display region is moving, and displaying the blood flow image when the movement stops.

However, as described above, in the ultrasonic diagnostic equipment, the two-dimensional distribution of a blood flow is displayed in the blood flow region formed in the B mode image display region in a color image, but while the blood flow display region is moving, transmission of ultrasonic waves is conventionally intermitted. Therefore, the blood flow display image during that time is not displayed, and the B mode image is only displayed. Therefore, there arises a problem that the blood flow state of a moving organ cannot be observed.

Further, there arises another problem that when the observation target of which blood flow state needs to be grasped move vigorously, if excess color display is performed, the target part of interest is difficult to be recognized visually in real time.

### SUMMARY OF THE INVENTION

The present invention is made in view of the above described circumstances, and has an object to provide ultrasonic diagnostic equipment and a method for processing a signal of ultrasonic diagnostic equipment capable of always optimally observing an observation target of which blood flow state needs to be grasped in a Doppler image in a Color Doppler mode or a Power Doppler mode in real time with simple processing at low cost.

Ultrasonic diagnostic equipment of the present invention is configured by including
ultrasonic pulse transmitting means transmitting an ultrasonic pulse to an inspection target,
ultrasonic echo signal receiving means receiving an ultrasonic echo signal of the ultrasonic pulse from the inspection target,
B mode image generating means generating a B mode image of the inspection target based on the ultrasonic echo signal received by the ultrasonic echo signal receiving means,
first region of interest setting means setting a first region of interest on the B mode image,
movement instructing means instructing movement of a predetermined set region on the B mode image,
second region of interest setting means setting a second region of interest in a movement destination of the set region by the movement instructing means,
Doppler image generating means generating a Doppler image by blood flow state information of the first and/or the second region of interest based on the ultrasonic echo signal received by the ultrasonic echo signal receiving means,
set region image generation control means controlling generation of the set region image by the moving state image generating means, and
image synthesizing means generating a synthesized image in which the B mode image, the Doppler image and the set region image are synthesized.

Other features and advantages of the present invention will become sufficiently apparent upon reading the following description.

### BRIEF DESCRIPTION OF THE DRAWINGS

Figs. 1 to 7 relate to an embodiment 1 of the present invention;
Fig. 1 is a block diagram showing a configuration of ultrasonic diagnostic equipment;
Fig. 2 is a flowchart showing a flow of processing of the ultrasonic diagnostic equipment in Fig. 1;
Fig. 3 is a first diagram explaining the processing in Fig. 2;
Fig. 4 is a second diagram explaining the processing in Fig. 2;
Fig. 5 is a third diagram explaining the processing in Fig. 2;
Fig. 6 is a fourth diagram explaining the processing in Fig. 2;
Fig. 7 is a fifth diagram explaining the processing in Fig. 2;
Figs. 8 to 34 relate to an embodiment 2 of the present invention;

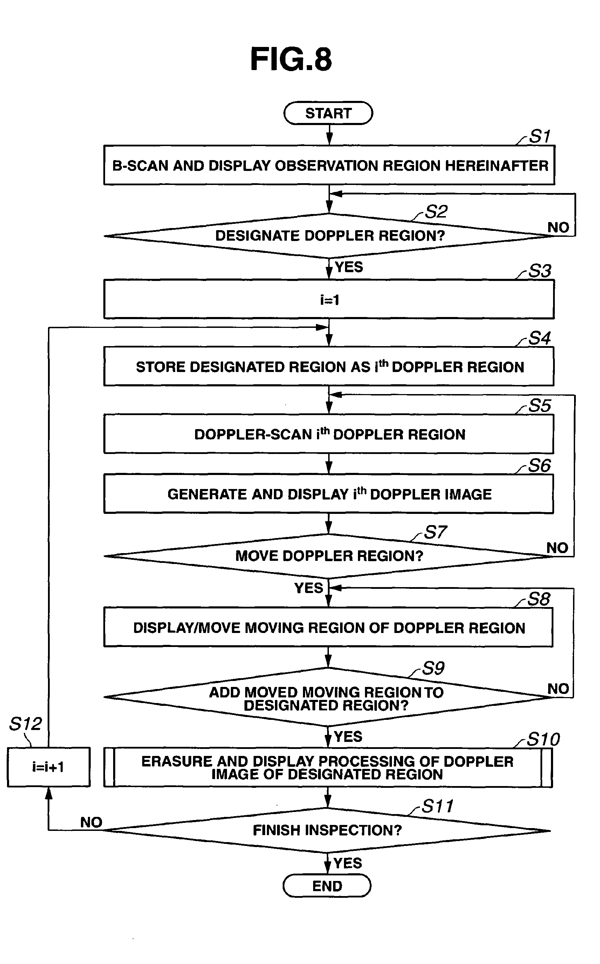

Fig. 8 is a flowchart showing a flow of the processing of ultrasonic diagnostic equipment;

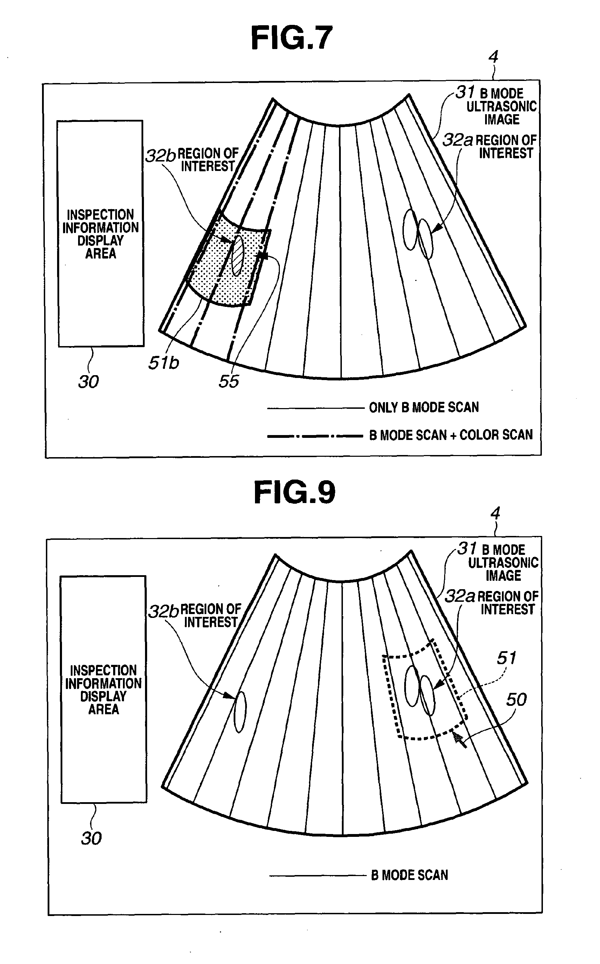

Fig. 9 is a first diagram explaining the processing of Fig. 8;

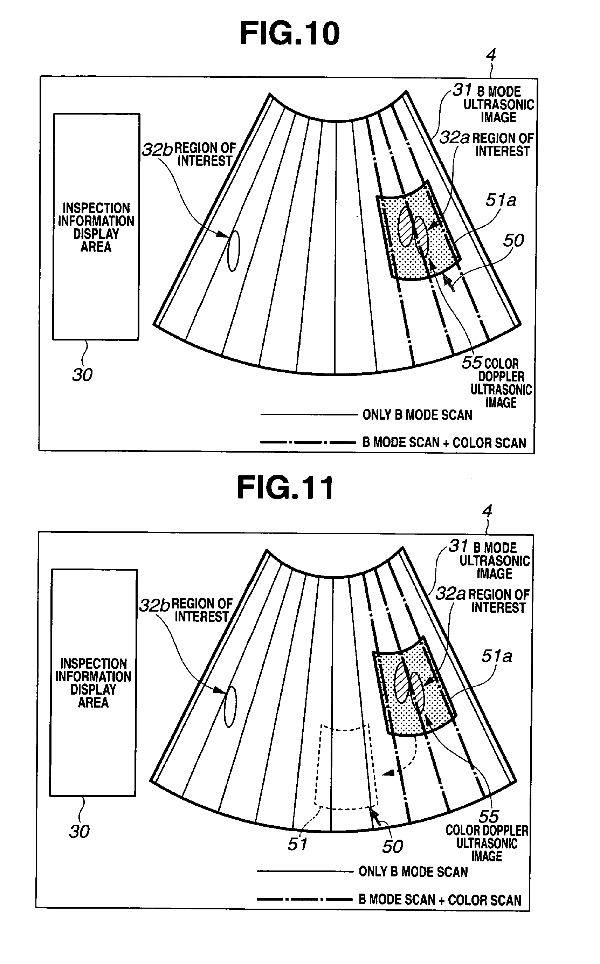

Fig. 10 is a second diagram explaining the processing of Fig. 8;
Fig. 11 is a third diagram explaining the processing of Fig. 8;

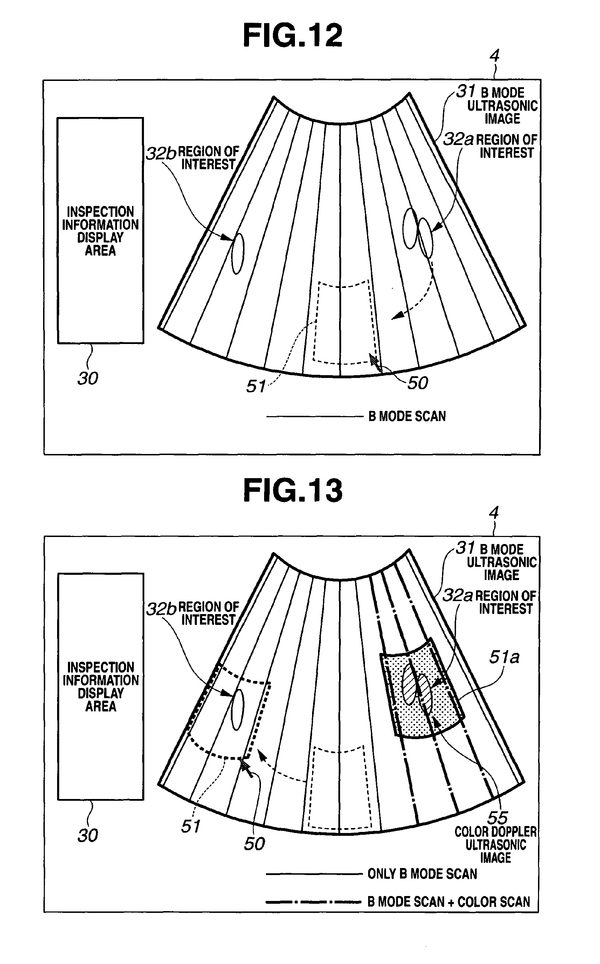

Fig. 12 is a fourth diagram explaining the processing of Fig. 8;
Fig. 13 is a fifth diagram explaining the processing of Fig. 8;

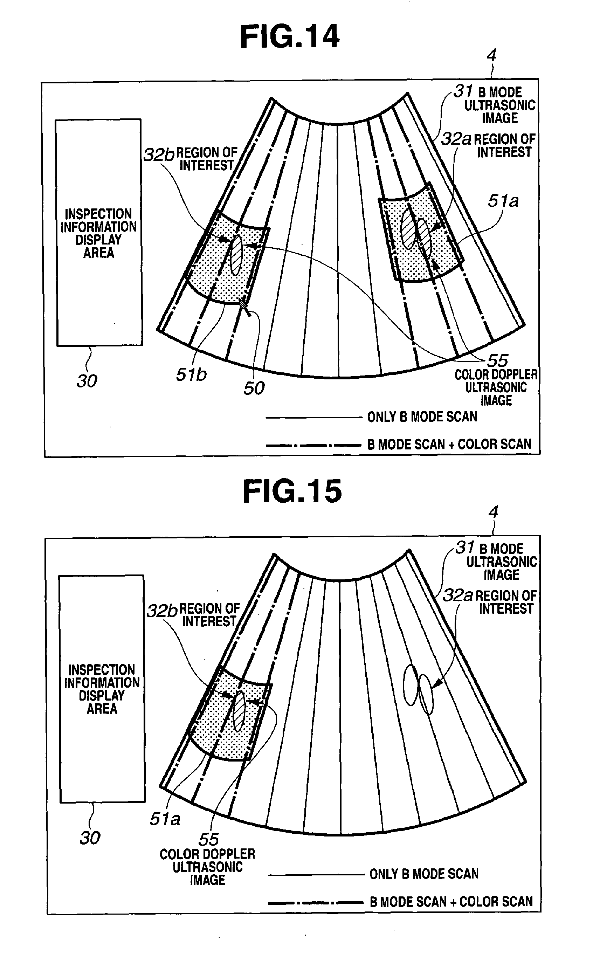

Fig. 14 is a sixth diagram explaining the processing of Fig. 8;
Fig. 15 is a seventh diagram explaining the processing of Fig. 8;

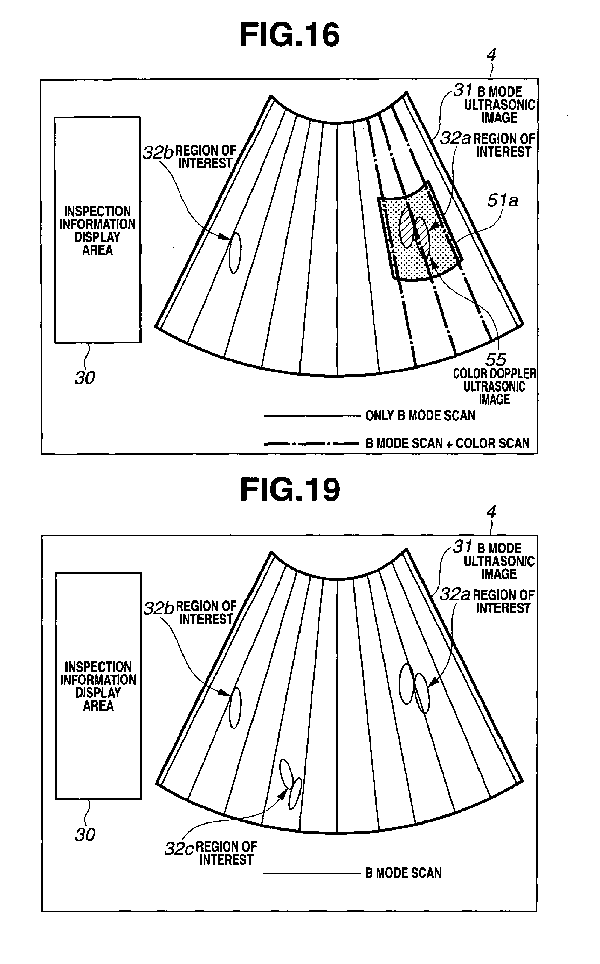

Fig. 16 is an eighth diagram explaining the processing of Fig. 8;

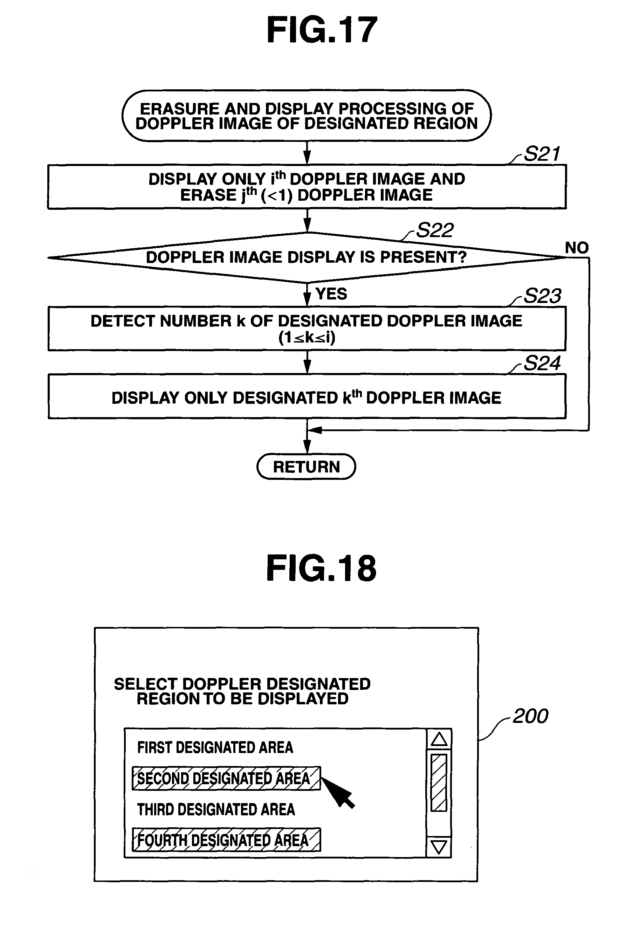

Fig. 17 is a flowchart showing a flow of erasure and display processing of a Doppler scan region image of a designated region of Fig. 8;
Fig. 18 is a first diagram explaining the processing of Fig. 17;
Fig. 19 is a second diagram explaining the processing of Fig. 17;

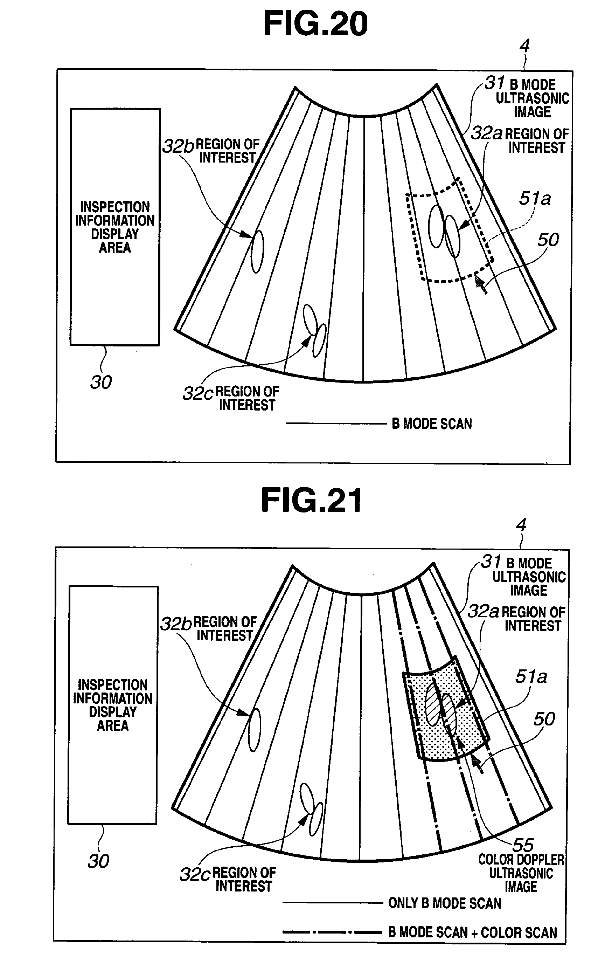

Fig. 20 is a third diagram explaining the processing of Fig. 17;
Fig. 21 is a fourth diagram explaining the processing of Fig. 17;

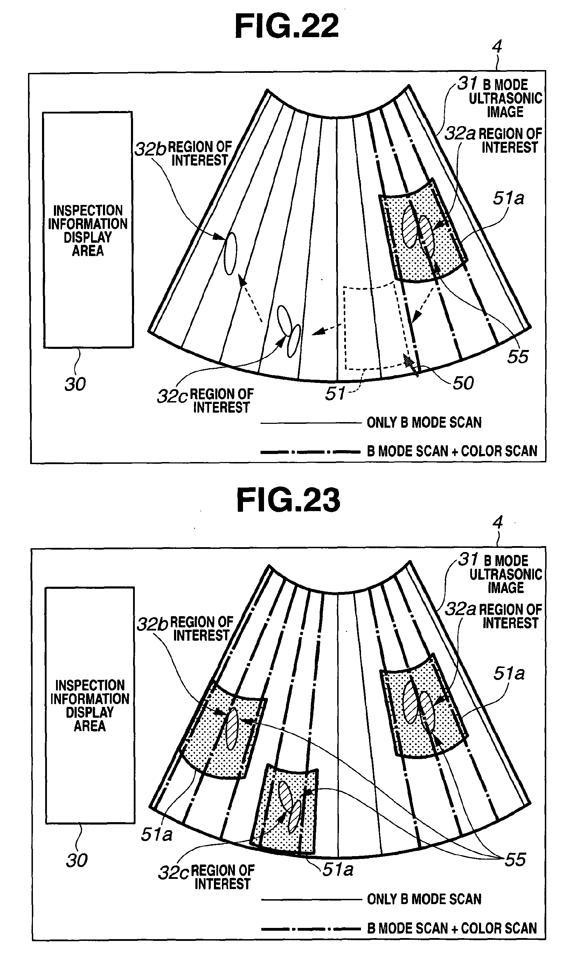

Fig. 22 is a fifth diagram explaining the processing of Fig. 17;
Fig. 23 is a sixth diagram explaining the processing of Fig. 17;

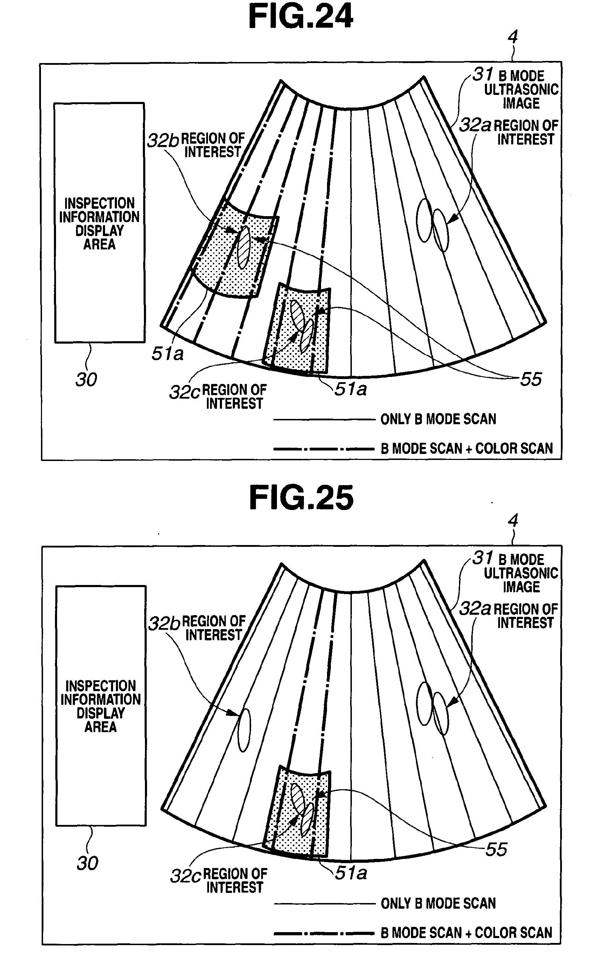

Fig. 24 is a seventh diagram explaining the processing of Fig. 17;
Fig. 25 is an eighth diagram explaining the processing of Fig. 17;

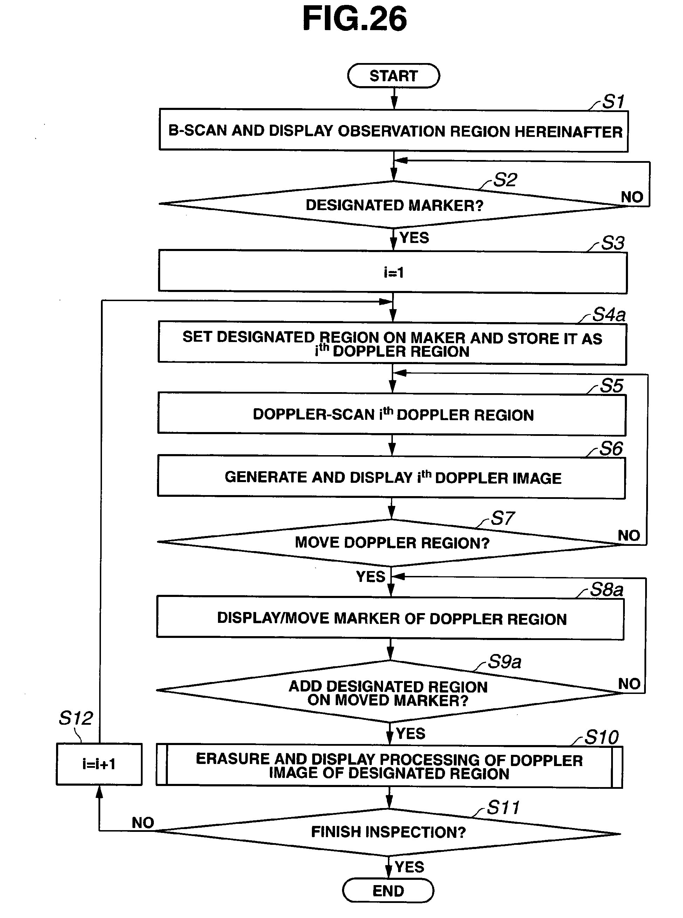

Fig. 26 is a flowchart showing a flow of a modified example of the processing of Fig. 8;

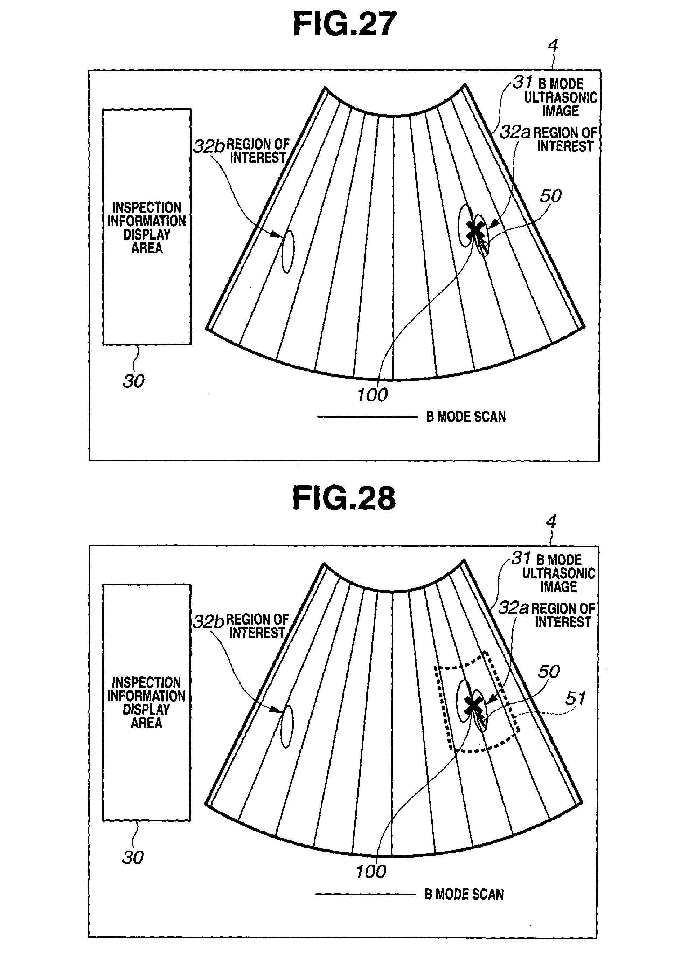

Fig. 27 is a first diagram explaining the processing of Fig. 26;
Fig. 28 is a second diagram explaining the processing of Fig. 26;

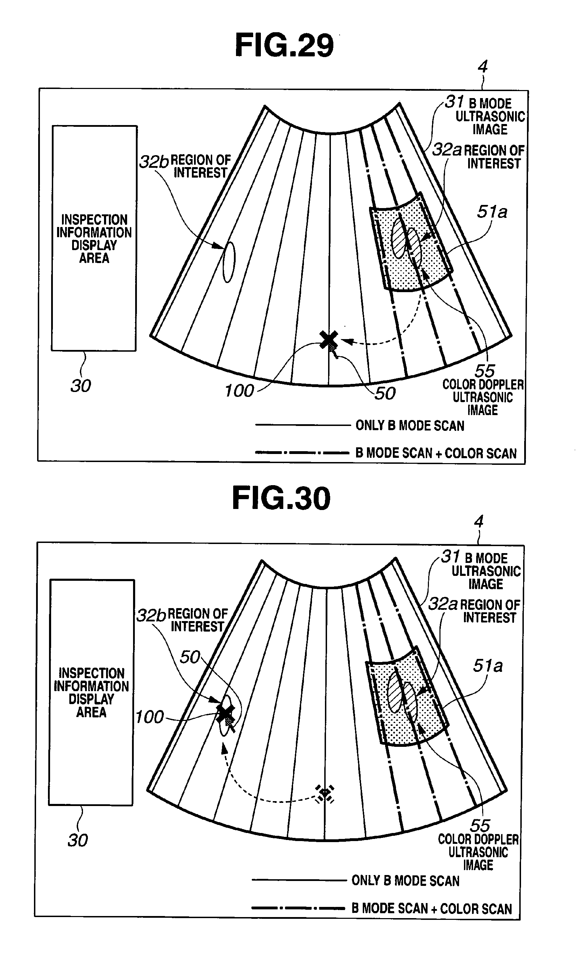

Fig. 29 is a third diagram explaining the processing of Fig. 26;
Fig. 30 is a fourth diagram explaining the processing of Fig. 26;

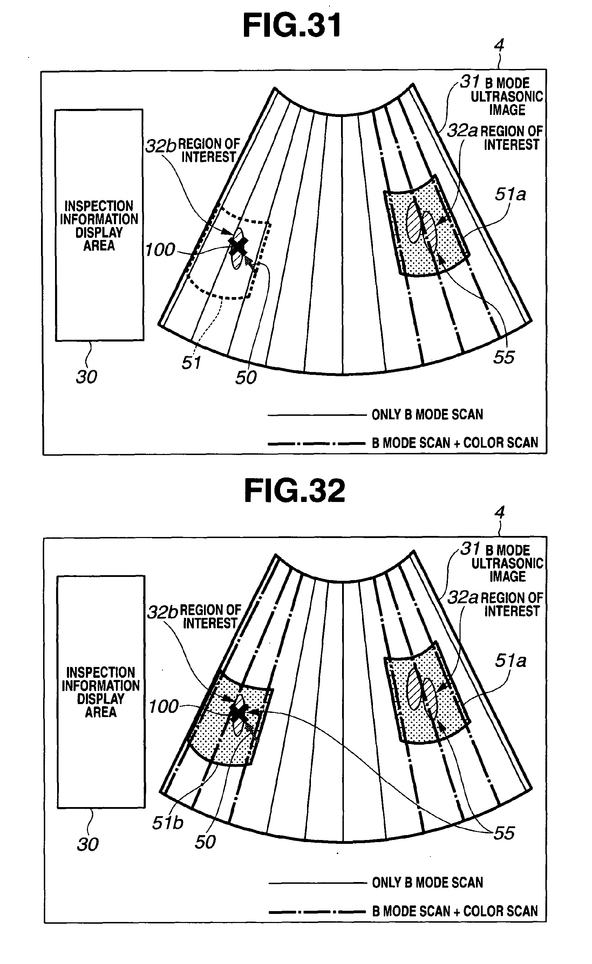

Fig. 31 is a fifth diagram explaining the processing of Fig. 26;
Fig. 32 is a sixth diagram explaining the processing of Fig. 26;

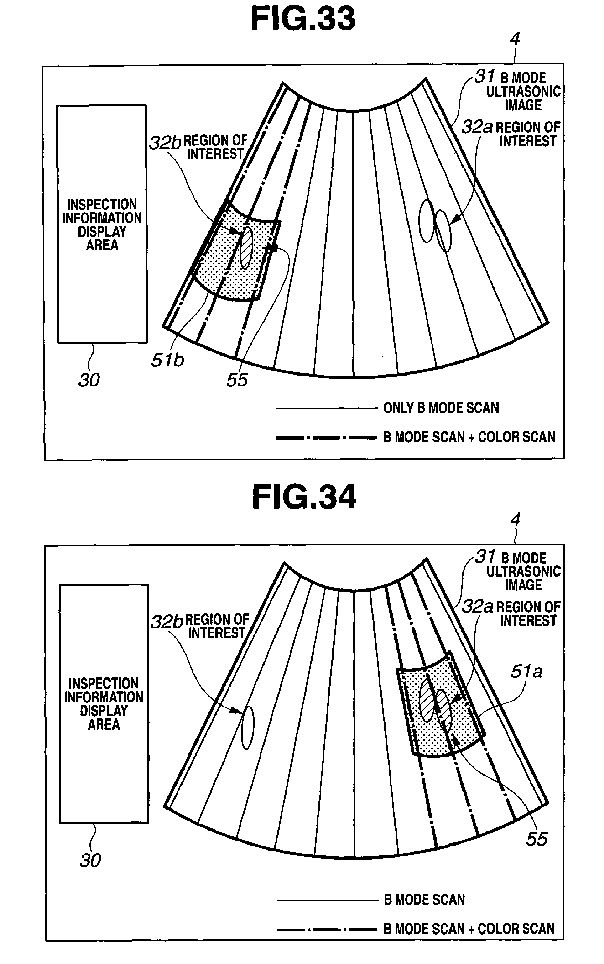

Fig. 33 is a seventh diagram explaining the processing of Fig. 26;
Fig. 34 is an eighth diagram explaining the processing of Fig. 26;

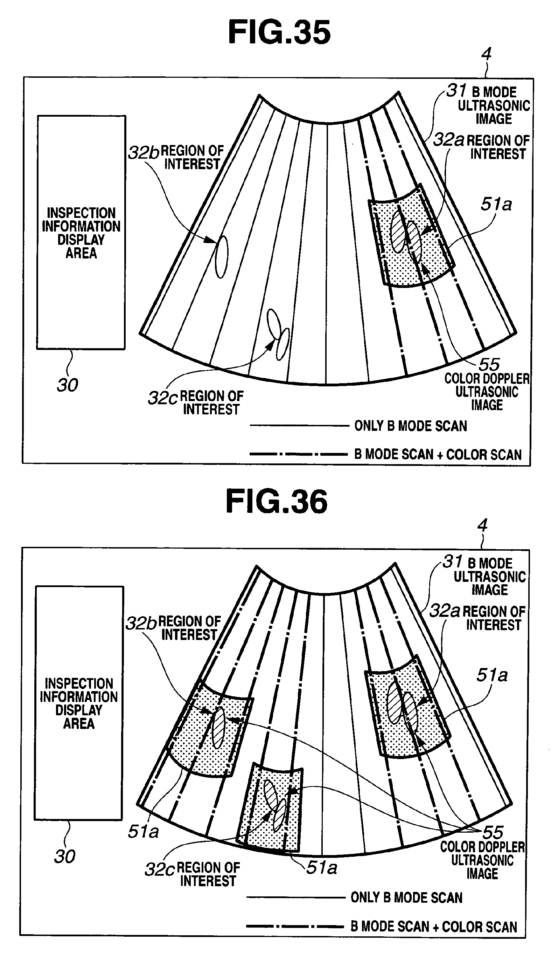

Fig. 35 is a diagram explaining a monitor screen of ultrasonic diagnostic equipment of an embodiment 3;
Fig. 36 is a diagram explaining the monitor screen of the ultrasonic diagnostic equipment of the embodiment 3;

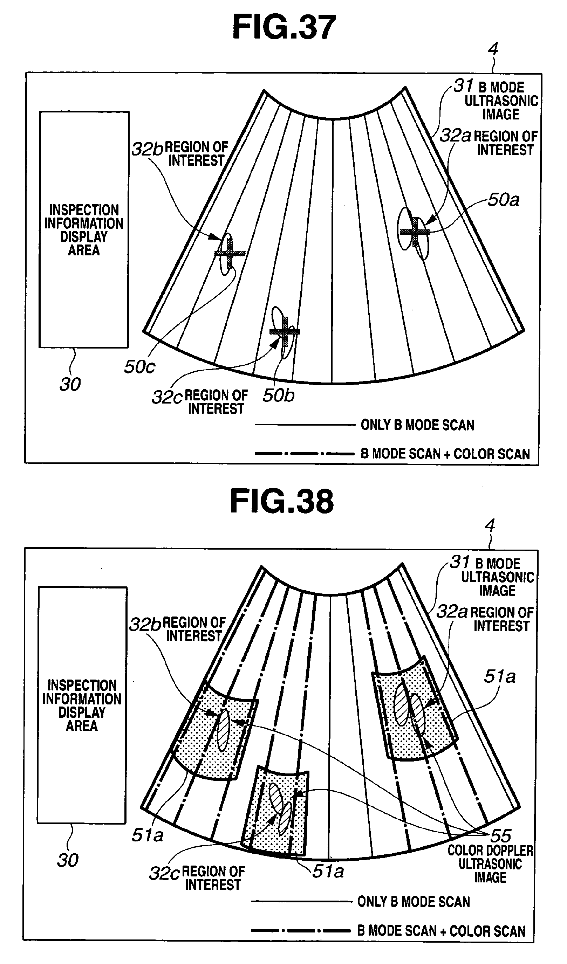

Fig. 37 is a diagram explaining the monitor screen of the ultrasonic diagnostic equipment of the embodiment 3;
Fig. 38 is a diagram explaining the monitor screen of the ultrasonic diagnostic equipment of the embodiment 3;

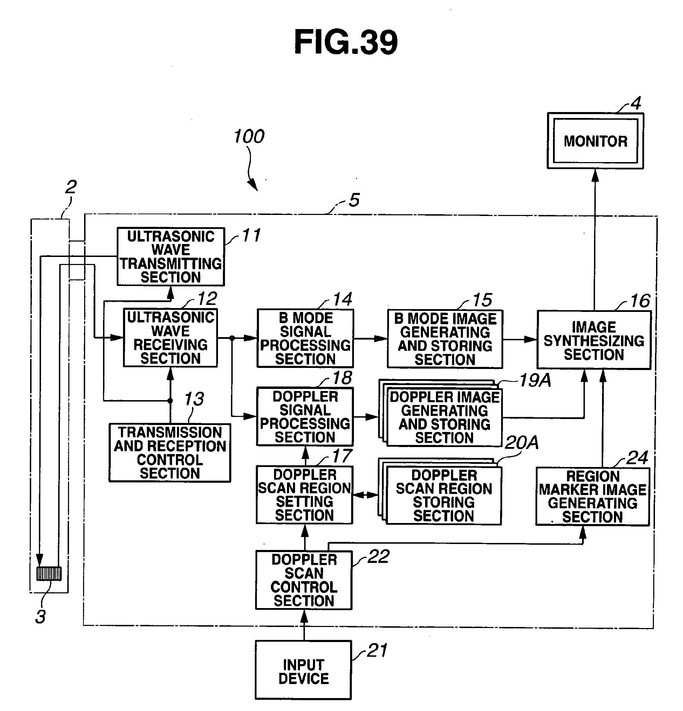

Fig. 39 is a block diagram showing a configuration of the ultrasonic diagnostic equipment of the embodiment 3;

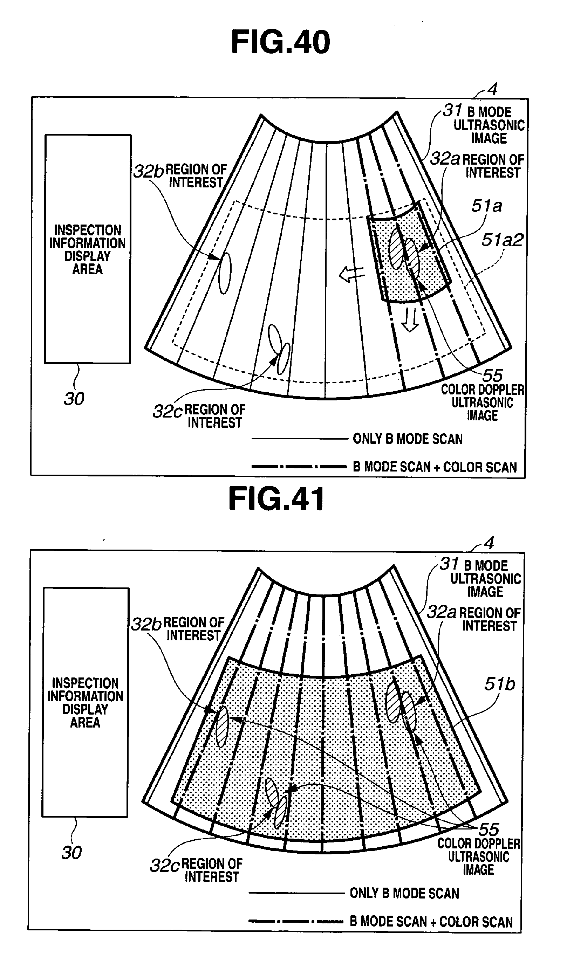

Fig. 40 is a diagram explaining a monitor screen in conventional ultrasonic diagnostic equipment; and
Fig. 41 is a diagram explaining the monitor screen in the conventional ultrasonic diagnostic equipment.

### DETAILED DESCRIPTION OF THE PREFERRED EMBODIMENTS

### [Embodiment 1]

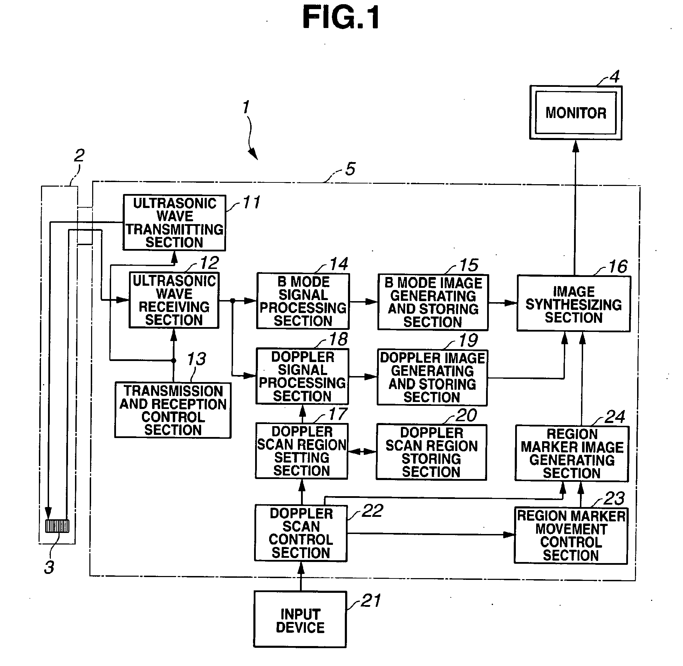

As shown in Fig. 1, ultrasonic diagnostic equipment 1 of the present embodiment is configured by including an ultrasonic probe 2 which is inserted into a luminal organ in a body cavity, and has, at a distal end portion, an ultrasonic transducer unit 3 transmitting and receiving ultrasonic waves to and from a part of interest such as an affected part in the luminal organ, and an image processing device 5 which ultrasonically drives the ultrasonic transducer unit 3 and displays an ultrasonic image on a monitor 4 by an ultrasonic echo signal.

The image processing device 5 is configured by including an ultrasonic wave transmitting section 11, an ultrasonic wave receiving section 12, a transmission and reception control section 13, a B mode signal processing section 14, a B mode image generating and storing section 15, an image synthesizing section 16, a Doppler signal processing section 18, a Doppler image generating and storing section 19, a Doppler scan region setting section 17, a Doppler scan region storing section 20, a Doppler scan control section 22, a region marker movement control section 23 and a region marker image generating section 24.

An input device 21 configured by a keyboard or, for example, a mouse or a trackball that is a pointing device is connected to the Doppler scan control section 22. The input device 21 configures movement instructing means in the present embodiment.

The ultrasonic wave transmitting section 11 is a drive section which ultrasonically drives the ultrasonic transducer unit 3, and configures ultrasonic pulse transmitting means in the present embodiment.

Further, the ultrasonic wave receiving section 12 is a receiving section which receives an ultrasonic echo signal from the ultrasonic transducer unit 3, and configures ultrasonic echo signal receiving means in the present embodiment.

The transmission and reception control section 13 is a control section which controls the timing of transmission and reception of the ultrasonic wave transmitting section 11 and the ultrasonic wave receiving section 12.

The B mode signal processing section 14 performs signal processing on an ultrasonic echo signal received by the ultrasonic wave receiving section 12 to generate a B mode image, creates the generated B mode image, and stores the generated B mode image in the B mode image generating and storing section 15, and configures B mode image creating means in the present embodiment.

The Doppler signal processing section 18 processes the ultrasonic echo signal received by the ultrasonic wave receiving section 12 by a Pulse Doppler method, generates a Doppler image expressing image data for displaying a CFM (Color Flow Mapping) image regarding blood flow dynamics, namely, a blood flow velocity, power of a Doppler signal, distribution of the blood flow velocity and the like, and stores the Doppler image in the Doppler image generating and storing section 19, and configures Doppler image generating means in the present embodiment.

The Doppler scan region setting section 17 sets a processing region of the Doppler image, which is generated by the Doppler signal processing section 18, on the B mode image generated by the B mode signal processing section 14, and configures first region of interest setting means and second region of interest setting means in the present embodiment.

The Doppler scan region storing section 20 stores region information of the processing region of the Doppler image set by the Doppler scan region setting section 17.

The Doppler scan control section 22 controls setting of the processing region of the Doppler image in the Doppler scan region setting section 17, and configures Doppler image region of interest designating means in the present embodiment.

The region marker image generating section 24 generates a Doppler scan region image on the B mode image of the processing region of the Doppler image set by the Doppler scan region setting section 17, and configures moving state image generating means in the present embodiment.

The region marker movement control section 23 controls movement of the Doppler scan region image generated by the region marker image generating section 24 on the B mode image, and configures set region image generation control means in the present embodiment.

The image synthesizing section 16 synthesizes the B mode image, the Doppler image and the Doppler scan region image and displays the same on the monitor 4, and configures image synthesizing means in the present embodiment.

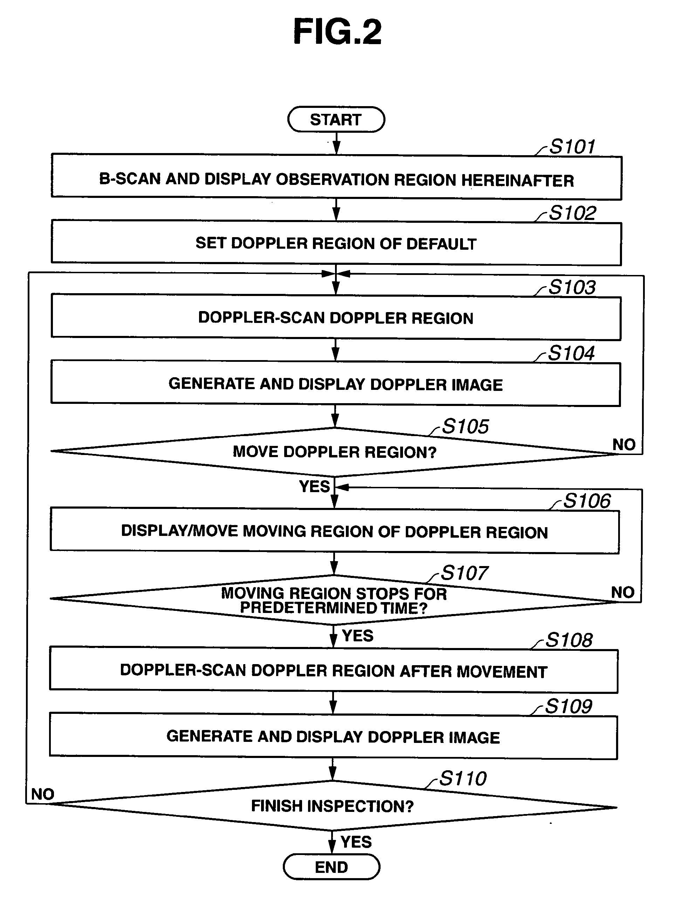

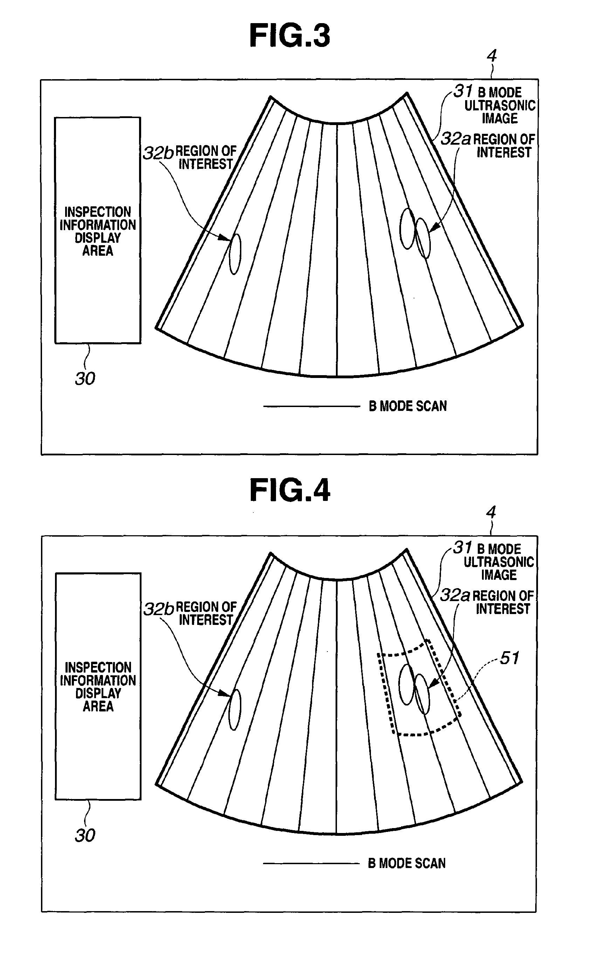

An operation of the present embodiment thus configured will be described by using a flowchart in Fig. 2 and explanatory diagrams in Figs. 3 to 7.

When an operator inserts the ultrasonic probe 2 into a luminal organ inside a body cavity and starts diagnosis, the ultrasonic diagnostic equipment 1 of the present embodiment scans an observation region of the luminal organ in a B mode as shown in Fig. 2, and displays a B mode ultrasonic image 31 as shown in Fig. 3 on the monitor 4 via the image synthesizing section 16 in step S101. The monitor 4 on which the B mode ultrasonic image 31 is displayed displays an inspection information area 30 displaying inspection information (patient information, inspection date and time and the like). Further, in the B mode ultrasonic image 31, for example, parts of interest 32a and 32b which are a plurality of regions of interest in which the operator who conducts diagnosis is interested are displayed.

Next, in step S102, the Doppler scan region setting section 17 sets a Doppler scan region 51 of default of a predetermined size stored in the Doppler scan region storing section 20 as shown in Fig. 4. At this time, scan of the ultrasonic image displayed on the monitor 4 is formed by only B mode scan.

Next, the Doppler signal processing section 18 processes the Doppler scan region 51 by a Pulse Doppler method based on the ultrasonic echo signal received by the ultrasonic wave receiving section 12 in step S 103, and generates a Doppler scan region image expressing image data for displaying a CFM (Color Flow Mapping) image regarding blood flow dynamics, namely, the blood flow velocity, power of the Doppler signal, distribution of the blood flow velocity and the like, and stores the Doppler scan region image in the Doppler image generating and storing section 19.

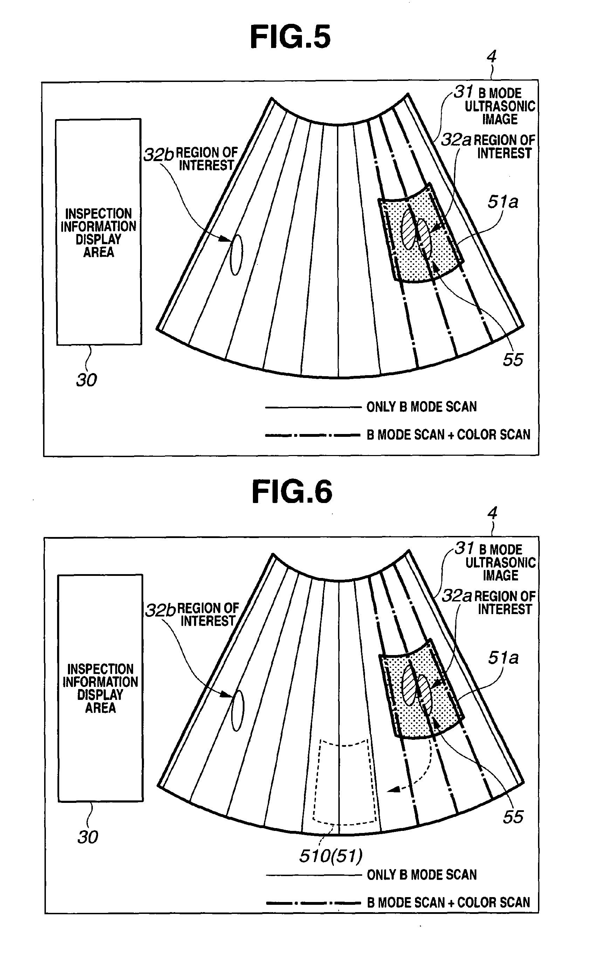

Subsequently, in step S104, the image synthesizing section 16 generates a synthesized image in which a Doppler scan region image 55 of a region of interest 51 a as a first region of interest that is the Doppler scan region 51 is superimposed on the B mode ultrasonic image 31, and displays the synthesized image on the monitor 4 as shown in Fig. 5.

Next, in step S105, it is determined whether or not a movement instruction of the Doppler scan region 51 is given by the input device 21 configured by, for example, a track ball by the operation of the operator, and when the movement instruction of the Doppler scan region 51 is not given, the flow returns to step S104. When the movement instruction of the Doppler scan region 51 is given, the flow goes to step S106.

Then, in step S106, as shown in Fig. 6, the region marker movement control section 23 controls the region marker image generating section 24, and moves a moving region frame image 510 based on an input signal of the input device 21 configured by, for example, a track ball, while displaying the synthesized image in which the Doppler scan region image 55 of the region of interest 51a is superimposed, and displaying the moving region frame image 510 showing the Doppler scan region 51 on the B mode ultrasonic image 31. At this time, Doppler scan is performed for displaying the Doppler scan region image 55 in real time.

In the conventional ultrasonic diagnostic equipment, only the moving region frame image 510 showing the Doppler scan region 51 is moved while the moving region frame image 510 is displayed on the B mode ultrasonic image 31, and the Doppler scan region image 55 of the region of interest 51a (of the part of interest 32a) cannot be observed (see Fig. 12).

Next, in step S107, the region marker movement control section 23 determines whether the operation of, for example, the track ball is stopped for a specified time, and when it determines that the operation of the track ball stops for a specified time, it judges that the moved Doppler scan region 51 is set as a designated region, and the flow goes to step S108. When the region marker movement control section 23 determines that the operation of the track ball does not stop for a specified time, the region marker movement control section 23 judges that the moved Doppler scan region 51 is not set as a designated region, and the flow returns to step S106.

When the operation of the track ball stops, for example, for a certain time (for example, one second) or more, in step S108, the Doppler signal processing section 18 performs Doppler-scan at the position of the Doppler scan region 51 that is a region of interest (ROI) after movement, and generates a Doppler scan region image expressing image data for displaying the CFM (Color Flow Mapping) image regarding blood flow dynamics, namely, the blood flow velocity, power of the Doppler signal, distribution of the blood flow velocity and the like, and stores the Doppler scan region image in the Doppler image generating and storing section 19.

Then, in step S109, the image synthesizing section 16 generates a synthesized image in which the Doppler scan region image 55 of the region of interest 51b as the second region of interest that is the Doppler scan region 51 is superimposed on the B mode ultrasonic image 31, and displays the synthesized image on the monitor 4, and the flow goes to step S110.

In step S 110, judgment is made in accordance with the input of the operator using the input device 21 configured by a keyboard, and when termination of the inspection (diagnosis) cannot be confirmed, the flow returns to step S103 and when the termination of the inspection (diagnosis) is confirmed, the processing is finished.

By returning to step S103, and repeating the above described processing S103 to S108, as shown in Fig. 7, the Doppler scan region image 55 of the initial Doppler scan region 51 of the movement destination is displayed, and at the same time, the synthesized image in which the Doppler scan region image 55 of the previous Doppler scan region 51 is erased is displayed on the monitor 4.

As above, according to the present embodiment, when the Doppler scan region image of the first region of interest 51a is displayed when the B mode image is displayed, even if the display region of the Doppler scan region image is moved by the moving region frame image 510, the display of the Doppler scan region image of the first region of interest 51a is kept, and therefore, B mode observation and Doppler observation can be continued. When the Doppler scan region 51 in the movement destination is fixed by the moving region frame image 510, the Doppler scan region image of a second region of interest 51 b is displayed by being switched from the Doppler scan region image of the first region of interest 51 a, and therefore, Doppler observation of the region of interest can be continuously performed.

Accordingly, in the present embodiment, the observation target of which blood flow state needs to be grasped can be always optimally observed in real time in a color image by the Power Doppler mode.

Conventionally, the software detects the position of the blood flow display region with a constant timing even while the blood flow display region is moving, and Doppler scan is performed at the location of the detected blood flow display region. Thus, a load is exerted on the software. Therefore, a CPU with a high performance is required, which results in increase in the cost of the equipment.

However, in the present embodiment, while the blood flow display region is moving, Doppler scan is always performed in the blood flow display region at the time of start of moving, and therefore, a load is not exerted on the software as compared with the conventional equipment, and less expensive CPU can be used by simple processing.

### [Embodiment 2]

Embodiment 2 is substantially the same as the embodiment 1. Therefore, only the point differing from the embodiment 1 will be described, and the explanation of the same components will be omitted by assigning the same reference numerals and characters to them.

An operation of the present embodiment will be described by using flowcharts in Figs. 8 and 17, and explanatory diagrams in Figs. 3 and 9 to 16.

When an operator inserts the ultrasonic probe 2 into a luminal organ in a body cavity and starts diagnosis, the ultrasonic diagnostic equipment 1 of the present embodiment performs B mode scan for an observation region of the luminal organ, and displays the B mode ultrasonic image 31 on the monitor 4 as in the embodiment 1 in step S 1 as shown in Fig. 8 (see Fig. 3). The monitor 4 on which the B mode ultrasonic image 31 is displayed displays the inspection information area 30 for displaying inspection information (patient information, inspection date and time and the like). Further, in the B mode ultrasonic image 31, for example, parts of interest 32a and 32b, which are a plurality of regions of interest in which the operator performing diagnosis is interested, are displayed.

When, for example, the operator designates, for example, the part of interest 32a to a Doppler region as a designated region by using the input device 21 in step S2, the Doppler scan control section 22 sets a parameter i to one. Then, the flow goes to step S3.

In step S3, the Doppler scan region setting section 17 sets the Doppler scan region 51 of a predetermined size including the part of interest 32a by a pointer 50 using the input device 21 by the operation of the operator as shown in Fig. 9. At this time, scan of the ultrasonic image displayed on the monitor 4 is formed by only the B mode scan.

Next, in step S4, the Doppler scan region setting section 17 stores the region information on the B mode image in the Doppler scan region 51 in the Doppler scan region storing section 20 as region information of the i^{th} region of interest, for example.

Subsequently, in step S5, the Doppler signal processing section 18 processes the i^{th} region of interest by a Pulse Doppler method based on the ultrasonic echo signal received by the ultrasonic wave receiving section 12, generates the i^{th} Doppler scan region image 55 expressing image data for displaying a CFM (Color Flow Mapping) image regarding the blood flow dynamics, namely, the blood flow velocity, power of the Doppler signal, distribution of the blood flow velocity and the like, and stores the i^{th} Doppler scan region image 55 in the Doppler image generating and storing section 19.

Subsequently, in step S6, the image synthesizing section 16 generates a synthesized image in which the i^{th} Doppler scan region image 55 of the i^{th} region of interest 51a is superimposed on the B mode ultrasonic image 31, and displays the synthesized image on the monitor 4 as shown in Fig. 10.

Next, in step S7, it is determined whether or not an instruction to move the Doppler scan region 51 is given by the pointer 50 using the input device 21 by the operation of the operator. When the instruction to move the Doppler scan region 51 is not given, the flow returns to step S5. When the instruction to move the Doppler scan region 51 is given, the flow goes to step S8.

In step S8, as shown in Fig. 11, the region marker movement control section 23 controls the region marker image generating section 24 to move the Doppler scan region 51 based on the pointer 50 using the input device 21 while displaying the synthesized image in which the i^{th} Doppler scan region image 55 of the i^{th} region of interest 51a is superimposed, and also displaying the Doppler scan region 51 on the B mode ultrasonic image 31.

In the conventional ultrasonic diagnostic equipment, the Doppler scan region 51 is moved while only the Doppler scan region 51 is displayed on the B mode ultrasonic image 31 as shown in Fig. 12, and observation of the i^{th} Doppler scan region image 55 in the i^{th} region of interest 51a (of the part of interest 32a) cannot be performed.

Next, in step S9, as shown in Fig. 13, it is determined whether or not the moved Doppler scan region 51 is added as a designated region by the instruction using the input device 21 by the operation of the operator. When the moved Doppler scan region 51 is not added as the designated region, the flow returns to step S8, and when the moved Doppler scan region 51 is added as the designated region, the flow goes to step S10.

Subsequently, in step S10, the image processing device 5 displays an image as shown in Fig. 14, 15 or 16 on the monitor 4 in accordance with the instruction using the input device 21 by the operation of the operator to execute "ERASURE AND DISPLAY PROCESSING OF DOPPLER SCAN REGION IMAGE OF DESIGNATED REGION" which will be described later, and the flow goes to step S11.

In step S11, termination of the inspection (diagnosis) is determined by the input by the operator using the input device 21. When the termination of the inspection (diagnosis) cannot be confirmed, i is incremented in step S12 to return to step S4, and the processing is finished when the termination of the inspection (diagnosis) is confirmed.

Next, "ERASURE AND DISPLAY PROCESSING OF DOPPLER SCAN REGION IMAGE OF DESIGNATED REGION" of the above described step S10 will be described by using a flowchart in Fig. 17.

In the erasure and display processing of the Doppler scan region image of the designated region, the Doppler scan control section 22 of the image processing device 5 displays only the i^{th} Doppler scan region image 55 of the i^{th} region of interest 51b, and erases a k^{th} Doppler scan region image 55 with k<i in step S21 as shown in Fig. 17.

Subsequently, the Doppler scan control section 22 determines whether or not an instruction to display the Doppler scan region image 55 other than the i^{th} Doppler scan region image 55 is given by the instruction using the input device 21 by the operation of the operator. When the instruction is not given, the Doppler scan control section 22 finishes the processing, and when the instruction is given, the flow goes to step S23.

In step S23, the Doppler scan control section 22 displays a Doppler image selection window 200 as shown in Fig. 18 on the monitor 4, waits for selection of number k (k is an integer which is one or more and less than or equal to i: k^{th} designated area) of the Doppler designated region which is stored in the Doppler scan region storing section 20 by using the input device 21 by the operation of the operator in the Doppler image selection window 200, and detects the number k.

When the Doppler scan control section 22 detects the number k, the Doppler scan control section 22 displays only the k^{th} Doppler scan region image of the k^{th} designated area designated in step S24, and finishes the processing.

The above described Fig. 14 shows the display example of the image when the first and second Doppler scan region images are designated, the above described Fig. 15 shows the display example of the image when only the first Doppler scan region image is designated, and the above described Fig. 16 shows the display example of the image when only the second Doppler scan region image is designated.

Figs. 19 to 25 show the display examples of the above described processing when more than two parts of interest, for example, three parts of interest 32a, 32b and 32c are present.

In the present embodiment, the region of the Doppler scan region image 55 is designated by the Doppler scan region 51, but the present invention is not limited to this.

Specifically, as shown in steps S4a, 8a and 9a in Fig. 26, a marker 100 may be used instead of the Doppler scan region 51. In concrete, as shown in Figs. 27 to 34, by moving the marker 100 and setting the Doppler scan region 51 of a predetermined size including the marker 100, the same operation as in the present embodiment can be obtained.

As described above, according to the present embodiment, as in the embodiment 1, in the case of displaying the Doppler scan region image when the B mode image is displayed, display of the Doppler scan region image is kept even if the display region of the Doppler scan region image is moved, and therefore, B mode observation and Doppler observation can be continued.

In the present embodiment, in addition to the above described effect, a plurality of Doppler scan region images can be displayed by being superimposed on the B mode image, and therefore, the effect of being able to observe and diagnose the blood flow state of a part of interest at the same time is provided.

Accordingly, in the present embodiment, as in the embodiment 1, the observation target of which blood flow state needs to be grasped can be always optimally observed in real time in a color image by the Power Doppler mode.

### [Embodiment 3]

Hereinafter, ultrasonic diagnostic equipment 100 according to a third embodiment of the present invention will be described with inclusion of comparison with the conventional ultrasonic diagnostic equipment. Figs. 40 and 41 are both diagrams explaining monitor screens in the conventional ultrasonic diagnostic equipment. In the conventional ultrasonic diagnostic equipment, the number of blood flow display regions 51 a (hereinafter, called a color display region) of which color image display (hereinafter, called color display) of the two-dimensional distribution of the blood flow can be made is only one in the region of the B mode image display 31. Therefore, in order to make color display of a plurality of target parts of interest 32a, 32b and 32c in the region of the B mode image display 31 in real time at the same time, there is no other method but to extend the color display region 51a including one part of interest 32a which is displayed in colors to be large as shown in Fig. 40, and designate the color display region 51b including all the plurality of target parts of interest 32a, 32b and 32c as shown in Fig. 41.

However, if the color display region 51 a is extended to be large, signal processing and the like increase, and the frame rate is reduced, that is, the display response reduces. Therefore, the method for extending the color display region 51 a is not suitable for the case in which the region of interest is desired to be observed at a high speed. Further, parts of no interest existing between the plurality of target parts of interest 32a and 32b or 32c are sometimes displayed in a color image. Then, it becomes not easy for the operator to recognize only the information of the necessary target part of interest.

The present embodiment is made in view of the above described problems, and has an object to provide ultrasonic diagnostic equipment capable of making color display of only a plurality of target parts of interest in the B mode image display region in real time.

Figs. 35 to 38 are diagrams explaining the monitor screen of the ultrasonic diagnostic equipment according to the embodiment 3 of the present invention. Figs. 35 and 36 display the screen on which color display of only one part of interest 32a in the region of the B mode image display 31 is made shown in Fig. 35 and the screen on which color display of three parts of interest 32a, 32b and 32c is made shown in Fig. 36 in the ultrasonic diagnostic equipment of the present embodiment by comparison. In order to designate a plurality of parts of interest by an operator, the operator designates them by using pointers 50a, 50b and 50c by an input device, for example, a mouse or the like as shown in Fig. 37. Then, as shown in Fig. 38, three color display regions 1a are formed around the pointers 50a, 50b and 50c, and color display of a plurality of parts of interest 32a, 32b and 32c in them is made in real time at the same time.

Further, in the ultrasonic diagnostic equipment of the present embodiment, if a region of interest is the region of interest which is designated once, that is, if it is the designated area, stop of color display of the designated area, or restart of the color display can be both performed instantly. Stop of display of the designated area and the like are designated by operating the screen similar to the display screen shown in Fig. 18.

In the ultrasonic diagnostic equipment of the present embodiment, color display of only a necessary part of interest can be made when the operator needs it. Therefore, color display of a plurality of target parts of interest can be simultaneously made while reduction in frame rate is prevented as much as possible. In the ultrasonic diagnostic equipment of the present embodiment, color display of the blood flow information of the region other than the region of interest is not made on the display screen, and therefore, the operator easily recognizes the information of the region of interest.

In the ultrasonic diagnostic equipment of the present embodiment, the concept of "movement of a region of interest" does not exist. In the ultrasonic diagnostic equipment of the present embodiment which can display a plurality of color display regions at the same time, a region of interest is newly added simply. Therefore, in the ultrasonic diagnostic equipment of the present embodiment, motion display or the like while the region of interest is moving is not necessary on the B mode image display screen.

Next, Fig. 39 is a block diagram showing a configuration of the ultrasonic diagnostic equipment 100 of the present embodiment. In Fig. 39, the same components as in Fig. 1 are assigned with the same reference numerals and characters, and the explanation of them will be omitted.

Unlike the ultrasonic diagnostic equipment 1 of the embodiment 1 shown in Fig. 1, in the ultrasonic diagnostic equipment 100 of the present embodiment, the region marker movement control section 23 is not required as shown in Fig. 39.

Unlike the ultrasonic diagnostic equipment 1 of the embodiment 1 shown in Fig. 1, the ultrasonic diagnostic equipment 100 of the present embodiment desirably has a plurality of Doppler scan region storing section 20A and a plurality of Doppler image generating and storing section 19A to make color display of a plurality of regions of interest in the B mode image display region in real time at the same time.

Specifically, the ultrasonic diagnostic equipment 100 of the present embodiment is ultrasonic diagnostic equipment having the region marker image generating section 24 for designating a Doppler scan region (color display region), the Doppler scan region setting section 17 for setting a Doppler scan region, a plurality of Doppler scan region storing sections 20A storing the position or region of the Doppler scan region, the transmission and reception control section 13 which controls transmitting and receiving means which transmits and receives ultrasonic waves to and from a Doppler scan region and obtains Doppler data, and transmission and reception of ultrasonic waves, the Doppler signal section 18 which processes Doppler data obtained in each Doppler scan region, a plurality of Doppler image generating and storing section 19A which generate and store Doppler images, and an image synthesizing section 16 which synthesizes a Doppler image onto a Doppler scan region on the B mode display image.

Markers are set by the region marker image generating section 24 respectively near a plurality of parts of interest which are designated by the Doppler scan control section 22 via the input device 21. At the same time, a plurality of Doppler scan regions are set by the Doppler scan region setting section 17, and are stored in the Doppler scan region storing section 20A. Subsequently, by the information of the Doppler scan region storing section 20A, one Doppler scan region is set in sequence from a plurality of Doppler scan regions. With respect to the selected one Doppler scan region, ultrasonic wave transmission and reception are performed via the signal processing section 18, the Doppler signal is transmitted to the Doppler signal processing section 18, and a Doppler image is generated and stored in one Doppler image generating and storing section 19A selected from a plurality of them. Next, one Doppler scan region is set from the unselected Doppler scan regions, and the Doppler scan is performed as described above, and the Doppler image is stored in another Doppler image generating and storing section 19A. The processing is performed repeatedly, and the Doppler images and the region marker images of all the designated parts of interest are synthesized in the image synthesizing section 16, and the synthesized image is displayed on the monitor 4.

The ultrasonic diagnostic equipment 100 of the present embodiment described above can always display a plurality of parts of interest on the B mode image display screen in a Doppler image by the Color Doppler mode or the Power Doppler mode in real time at the same time.

In this invention, different embodiments in a wide range can be obviously configured based on the invention without departing from the spirit and scope of the invention. The invention is not restricted by the specified embodiments except that the invention is limited by the accompanying claims.

## Claims

1. Ultrasonic diagnostic equipment, comprising:
ultrasonic pulse transmitting means transmitting an ultrasonic pulse to an inspection target;
ultrasonic echo signal receiving means receiving an ultrasonic echo signal of the ultrasonic pulse from the inspection target;
B mode image generating means generating a B mode image of the inspection target based on the ultrasonic echo signal received by the ultrasonic echo signal receiving means;
first region of interest setting means setting a first region of interest on the B mode image;
movement instructing means instructing movement of a predetermined set region on the B mode image;
second region of interest setting means setting a second region of interest in a movement destination of the set region by the movement instructing means;
Doppler image generating means generating a Doppler image by blood flow state information of the first and/or the second region of interest based on the ultrasonic echo signal received by the ultrasonic echo signal receiving means;
moving state image generating means generating a set region image showing a moving state of the set region based on the movement instructing means;
set region image generation control means controlling generation of the set region image by the moving state image generating means; and
image synthesizing means generating a synthesized image in which the B mode image, the Doppler image and the set region image are synthesized.

2. The ultrasonic diagnostic equipment according to claim 1,
wherein the Doppler image generating means
continuously generates at least the Doppler image of the first region of interest while the set region is moving, and erases the Doppler image of the first region of interest when the second region of interest of the movement destination is set, and generates the Doppler image of the second region of interest, and
the image synthesizing means
synthesizes the Doppler image of the second region of interest which is continuously generated onto the B mode image and the set region image.

3. The ultrasonic diagnostic equipment according to claim 1,
wherein the Doppler image generating means
continuously generates at least the Doppler image while the set region is moving, and
the image synthesizing means
synthesizes the continuously generated Doppler image onto the B mode image and the set region image.

4. The ultrasonic diagnostic equipment according to any one of claims 1, 2 and 3, further comprising:
Doppler image region of interest designating means designating one or a plurality of regions of interest of the Doppler image which is synthesized by the image synthesizing means.

5. A method for processing a signal of ultrasonic diagnostic equipment, comprising:
an ultrasonic pulse transmitting step of transmitting an ultrasonic pulse to an inspection target;
an ultrasonic echo signal receiving step of receiving an ultrasonic echo signal of the ultrasonic pulse from the inspection target;
a B mode image generating step of generating a B mode image of the inspection target based on the ultrasonic echo signal received in the ultrasonic echo signal receiving step;
a first region of interest setting step of setting a first region of interest on the B mode image;
a movement instructing step of instructing movement of a predetermined set region on the B mode image;
a second region of interest setting step of setting a second region of interest in a movement destination of the set region by the movement instructing step;
a Doppler image generating step of generating a Doppler image by blood flow state information of the first and/or the second region of interest based on the ultrasonic echo signal received in the ultrasonic echo signal receiving step;
a moving state image generating step of generating a set region image showing a moving state of the set region based on an instruction in the movement instructing step;
a set region image generation control step of controlling generation of the set region image by the moving state image generating step; and
an image synthesizing step of generating a synthesized image in which the B mode image, the Doppler image and the set region image are synthesized.

6. The method for processing a signal of ultrasonic diagnostic equipment according to claim 5,
wherein in the Doppler image generating step,
at least the Doppler image of the first region of interest is continuously generated while the set region is moving, and when the second region of interest in the movement destination is set, the Doppler image of the first region of interest is erased, and the Doppler image of the second region of interest is generated, and
in the image synthesizing step,
the Doppler image of the second region of interest which is continuously generated is synthesized onto the B mode image and the set region image.

7. The method for processing a signal of ultrasonic diagnostic equipment according to claim 5,
wherein in the Doppler image generating step,
at least the Doppler image is continuously generated while the set region is moving, and
in the image synthesizing step,
the Doppler image continuously generated is synthesized onto the B mode image and the set region image.

8. The method for processing a signal of ultrasonic diagnostic equipment according to any one of claims 5, 6 and 7, further comprising:
a Doppler image region of interest designating step of designating one or a plurality of regions of interest of the Doppler image which is synthesized in the image synthesizing step.
